(19) Europäisches Patentamt
European Patent Office
Office européen des brevets

(11) Publication number: **0 237 983 B1**

(12) **EUROPEAN PATENT SPECIFICATION**

(45) Date of publication of patent specification: **20.05.92**

(51) Int. Cl.[5]: **C12P 17/02**, C12P 41/00

(21) Application number: **87103684.4**

(22) Date of filing: **13.03.87**

(54) **Process for the biotechnological preparation of L (-)-carnitine chloride.**

(30) Priority: **14.03.86 IT 1976286**

(43) Date of publication of application:
**23.09.87 Bulletin 87/39**

(45) Publication of the grant of the patent:
**20.05.92 Bulletin 92/21**

(84) Designated Contracting States:
**AT BE CH DE ES FR GB LI NL SE**

(56) References cited:
**US-A- 3 135 788**
**US-A- 4 259 249**

(73) Proprietor: **ISTITUTO GUIDO DONEGANI S.p.A.**
**Via Caduti del Lavoro**
**I-28100 Novara(IT)**

(72) Inventor: **Francalanci, Franco**
**7, Via G. Ferraris**
**I-28100 Novara(IT)**
Inventor: **Ricci, Marco**
**5, Via Brescia**
**I-28100 Novara(IT)**
Inventor: **Cesti, Pietro**
**51, Via Torino**
**I-28069 Trecate Novara(IT)**
Inventor: **Venturello, Carlo**
**135, Via XXIII Marzo**
**I-28100 Novara(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 237 983 B1

## Description

The present invention relates to a process for the preparation of L(-)-carnitine chloride having formula:

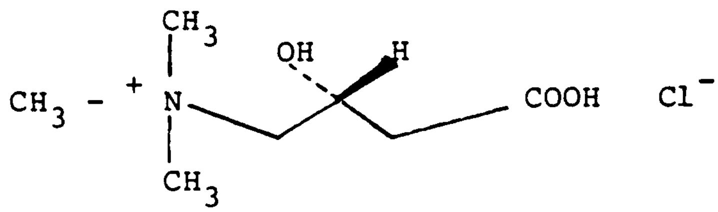

(I)

through a biotechnological preparation method.

More particularly, the present invention relates to a process for the preparation of L(-)-carnitine chloride based on the reaction of trimethylamine with an alkaline salt of R(+)-3,4-epoxybutyric acid which, in turn, is obtained through two successive reactions of enzymatic hydrolysis reactions of particular esters of aforesaid racemic 3,4-epoxybutyric acid.

As known, carnitine (also defined as $\beta$-hydroxy-$\gamma$-trimethylaminobutyric acid) contains a centre of asymmetry in $\beta$ position and, therefore, two stereoisomers may exist, usually defined as form D and form L, antipodes or optical enantiomers.

L(-)-carnitine, obtained in the form of chloride, according to the present invention, takes a prominent part in the field of the human metabolism and in the transfer of the fatty acids. D(+)-carnitine, on the contrary, is an inhibiting agent competitive with respect to L(-)-carnitine-acyltransferase and may cause the lowering of the level of L(-)-carnitine present in the cardiac tissue, see Fritz, I.B., Schultz, S. K. J. Biol. Chem. (1965) 240 2188; Roe, C.R., Bohan T.P. Lancet (1982) 1411.

The most ordinary therapeutic uses of L(-)-carnitine are, therefore, as eutrophic agent and as cardioprotecting agent in the treatment of myocardic ischemias, angina pectoris and myocardium scleroris.

Some processes are known for the carnitine preparation by synthesis, most of them, however, lead to obtain carnitine in the racemic form; therefore, an operating stage is required, aiming at separating the racemic mixture into the two single optical antipodes. Therefore, aforesaid processes need expensive reactants that are optically active such as, for instance, dibenzoyltartaric acid, caomphoric acid, mandelic acid, etc. Moreover, they require careful control of the reaction conditions and make several crystallization stages necessary; therefore, they prove to be complicated and consequently burdensome from the economic and operating point of view, in particular from the industrial point of view. (See European Patent Application EP 141.408; French Patent 1.466.696 and British Patent G.B. - A - 2.131.049).

A process was also described for the L(-)-carnitine synthesis, starting from an optically active compound such as D-mannitol (see European Patent Application EP 60.595). Such process, although it does not foresee any separation stage, requires, however, a great deal of steps and the use of expensive and potentially dangerous reactants such as lead tetraacetate.

Some microbiologic processes are also known, leading to L(-)-carnitine starting from prochiralic substrates, such as alkyl chloroacetoacetates, crotonbetaines or butyrobetaines (see Belgian Patent BE 898.396; European Patent Application EP 122.794; French Patent Application FR 2.485.564).

Such processes present the drawback to require bulky reaction volumes and are characterized by low yields and by difficulties in purifying the products.

Therefore the necessity was felt to have a process at disposal that could be carried out industrially, allowing the preparation of L(-)-carnitine in a simple, efficient and economic way.

Therefore an object of the present invention is to make the preparation of L(-)-carnitine chloride possible, according to a process simple from an operating point of view and advantageous from an industrial point of view.

The Applicant has now found that this and still other objects may be reached by means of a biotechnological process based on the reaction of trimethylamine with an alkaline salt of R(+)-3,4-epoxybutyric acid, the latter being obtained through two successive reactions of enzymatic hydrolysis of particular esters of aforesaid racemic 3,4-epoxybutyric acid. Then the obtained product is treated with HCl.

In practice, in the aforesaid two reactions of enzymatic hydrolysis use is made of two distinct enzymes, which are capable of hydrolyzing respectively as follows:

a) in a selective way, enantiomer S(-) of aforesaid racemic ester and

b) enantiomer R(+) of ester itself.

Therefore, the object of the present invention is to provide a process for the biotechnological preparation of L(-)-carnitine chloride having formula (I), which process consists in:

a) reacting a racemic ester of (R,S)-3,4-epoxybutyric acid having formula (II):

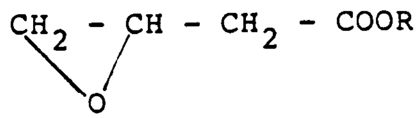

(II)

wherein R represents a $C_1$-$C_{10}$ alkyl group or a benzyl group, with an enzyme or with a microorganism producing said enzyme, capable of hydrolyzing asymmetrically and in a prevalent way enantiomer S(-), under pH conditions controlled by means of alkalis and in separating said enantiomer S(-) according to usual techniques from non-reacted ester (II) which is substantially present in the R(+) form;

b) reacting the R(+) form of ester (II), thus obtained, with an enzyme capable of hydrolyzing said R(+) form quantitatively, under pH conditions controlled by means of alkalis, to obtain a salt of 3,4-epoxybutyric acid having formula (III):

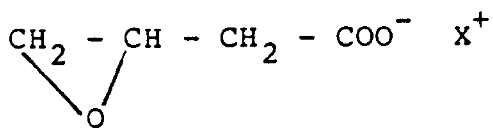

(III)

wherein X is a cation such as Na, K or Li, substantially in the R(+) form and

c) reacting said R(+) form of the salt having formula (III) with trimethylamine in a molar excess and, after having removed said excess, by means of hydrochloric acid, obtaining L(-)-carnitine chloride having formula (I) :

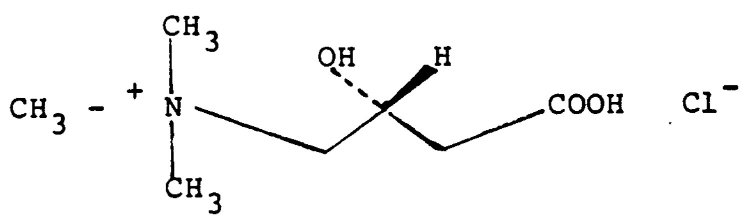

(I)

The racemic esters having formula (II) are per se known compounds and may be prepared according to usual processes, such as, for instance:

1) 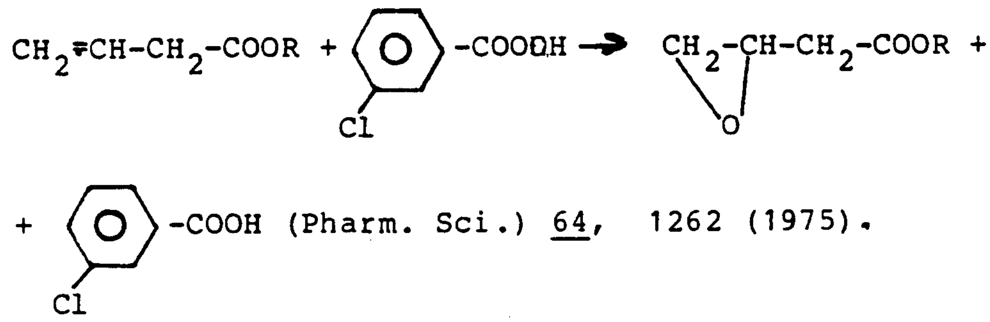

(Pharm. Sci.) 64, 1262 (1975).

2) 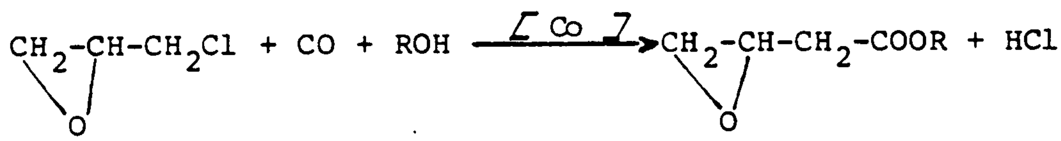


(J. Org. Chem. 32,3888).

As above mentioned, the racemic esters having formula (II) are reacted with enzymes, which may be produced by microorganisms or may be of animal origin, which enzymes are capable of hydrolyzing selectively enantiomer S(-), letting the enantiomer in the R(+) form substantially unaltered (asymmetric hydrolysis).

To this purpose use may be made of hydrolytic enzymes, of different origin, which may be found on the market; among them, the following ones, as defined hereinafter, proved to be particularly active.

| ENZYME | ORIGIN | PRODUCER |
|---|---|---|
| Steapsin | swine pancreas | SIGMA Chem. Co. St. Louis - USA |
| Pancreatin | " " | UNIBIOS - Trecate (ITALY) |
| Lipase from Candida cylindracea | *Candida cylindracea* | SIGMA Chem. Co. St. Louis - USA |
| Esterase from swine liver | swine liver | SIGMA Chem. Co. St. Louis - USA |

As above mentioned use may be made of microorganisms producing hydrolytic enzymes as well.

To this purpose use may be made of all the microorganisms, provided they may produce enzymes capable of hydrolyzing asymmetrically the racemic esters having formula (II).

Among these microorganisms the following ones proved to be particularly efficient:

| | | |
|---|---|---|
| Pseudomonas Fragi | IFO | 3458 |
| Bacillus subtilis | ATCC | 6633 |
| Rodotorula minuta | IFO | 0879 |
| Candida cylindracea | ATCC | 14830 |
| Arthrobacter simplex | IFO | 3530. |

The first reaction of asymmetric hydrolysis of the racemic esters having formula (II) is carried out by stirring strongly the mixture consisting of the racemic ester and of an aqueous solution of the raw of purified enzyme. Alternatively the aqueous solution of the enzyme may be replaced with a culture broth containing the microorganism , or with its filtrate or with its concentrate or with suspensions of the microorganism cells as well.

Alternatively, the enzyme may be used as immobilized on substrates of different nature, selected according to the prior art concerning this field. Use is made of enzyme amounts ranging between about 0.03% and 10% by weight, based on the racemic ester (II).

The first hydrolysis according to the invention may be carried out at a temperature ranging between about 5°C and 60°C, preferably between 10°C and 30°C, keeping the pH values between 5 and 9, preferably between 6 and 8, as the enzymes show their highest activity at these values.

The pH of the reaction medium is kept constant by using a Na or K buffer solution or by neutralization of the formed acidity by means of an alkali base such as, for instance, NaOH, KOH, LiOH, $CaCO_3$, etc.

The concentration of starting racemic ester (II) in the reaction mixture may range between 1% and 20% by weight. The reaction time of asymmetric hydrolysis may range between about 5 and 72 hours, according to the specific activity of the used enzyme, or to the desired conversion.

When the reaction of asymmetric hydrolysis is over, non-reacted ester (II), rich in isomer R(+), may be extracted from the reaction mixture by using a solvent immixible with water such as, for instance, methylene chloride, toluene, ligroine, ethyl ether etc. The ester thus obtained may be purified according to usual techniques such as, for instance, distillation or column chromatography.

The non-reacted ester having formula (II), substantially in the R(+) form, cannot be hydrolyzed easily be means of usual chemical methods, because of the high reactivity of the oxyrane ring, under the reaction conditions. According to the present invention, on the contrary, has been found that such hydrolysis may be carried out in a simple way, by using selected hydrolytic enzymes which are capable of hydrolyzing ester R(+) (II) quantitatively to obtain a salt of 3,4-epoxybutyric acid, substantially in the R(+) form.

Use may be made of commercial hydrolytic enzymes; among them the following ones proved to be particularly efficient.

| ENZYME | ORIGIN | PRODUCER |
|---|---|---|
| Subtilisina BPN' | Bacillus amyloliquefaciens | SIGMA Chem. Co. St. Louis - USA |
| Subtilisina Carlsberg | Bacillus subtilis | " " " " |
| Alcalase® | Bacillus Licheni= formis | NOVO Industri A/S DENMARK |

The aforesaid second reaction of hydrolysis of non-reacted ester (II) is carried out by stirring strongly the mixture consisting of non-reacted ester and of an aqueous solution of the raw or purified enzyme.

Alternatively the enzyme may be used as immobilized on substrates of different nature selected according to the prior art concerning this field.

Use is made of enzyme amounts ranging between about 0.5% and 30% by weight referred to ester R-(+) (II).

The hydrolysis according to the invention may be carried out at a temperature ranging between about 5 °C and 60 °C, preferably between 10 °C and 40 °C, keeping the pH between 5 and 10, preferably between 6 and 9. The pH in the reaction medium is kept constant by using a Na or K buffer solution or by neutralization of the formed acidity, by means of an alkali base such as, for instance, NaOH, KOH, LiOH. The concentration of non-reacted ester R(+) (II) in the reaction mixture, may range between about 1% and 20% by weight. The reaction time of hydrolysis may range between about 5 and 72 hours, according to the specific activity of the used enzyme.

At the end of the reaction, the resulting mixture is washed with a solvent immixible with water such as, for instance, methylene chloride, toluene, ligroine, ethyl ether, etc.

An aqueous solution of trimethylamine in excess is added to the aqueous phase containing an alkaline salt of 3,4-epoxybutyric acid having formula (III), substantially in the R(+) form, and the resulting mixture is stirred at a temperature ranging between about 10 °C and 60 °C, preferably between 40 °C and 50 °C, for a period of time ranging between about 1 and 12 hours.

The concentration of the trimethylamine solution may range between about 0.1 and 5.5 moles/litre; the trimethylamine is used in amounts ranging between about 1.5 and 3 moles per mole of salt of 3,4-epoxybutyric acid (III).

At the end of the reaction, water and non-reacted trimethylamine are removed by distillation.

NMR analysis of the residue in $D_2O$, shows exclusively the presence of the compound having formula (IV), i.e. of L(-)-carnitine as internal salt:

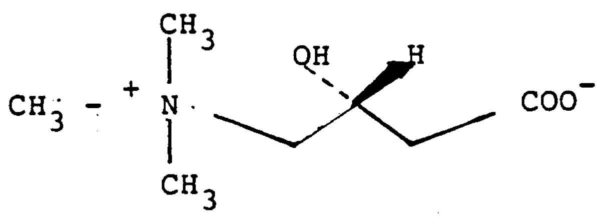

(IV)

The residue obtained after having removed the water and trimethylamine, is treated with aqueous hydrochloric acid, till an aqueous solution of L(-)-carnitine chloride having formula (I) is obtained.

From said aqueous solution, by evaporation of the water and then by crystallization of the residue, according to known techniques, finally L(-)-carnitine chloride having formula (I) is obtained.

From L(-)-carnitine chloride thus obtained, by means of methods that are also known, other derivatives of the L(-)-carnitine, such as salts of inorganic acids (HBr etc.) or organic acids (oxalic acid etc.) may be obtained.

A few examples will be given hereinafter by way of illustration but not of limitation of the invention.

The following abbreviations were used:

- $Eu(hfc)_3$ - europium tris[3-(heptafluoropropylhydroxymethylene)-d-camphorate].
- e.e. = enantiomeric excess.

EXAMPLE 1

Stage a)

45 ml of a buffer solution consisting of Na phosphate at pH = 7, 10 g of isobutyl (R,S)-3,4-epoxybutyrate (63.3 mmoles) and 640 mg of steapsin enzyme (lipase coming from swine pancreas, sold by the firm SIGMA Chem. Co. USA having a protein content of 35% and an activity equal to 35-70 units per mg of protein) were added to 45 ml of a 0.1 M solution of KCl. The mixture was strongly stirred at 20 °C, and the pH was kept constant at value 7 by addition of an aqueous solution of 5N NaOH. After 22 hours (conversion of 3,4-epoxybutyrate equal to 65%), the residual ester was recovered from the reaction mixture by extraction with methylene chloride and afterwards purified by distillation. Thus one recovered 3.3 of isobutyl R(+)-3,4-epoxybutyrate. NMR analysis at 300 MHz, in the presence of $Eu(hfc)_3$ (0.05 moles per mole of ester) showed an e.e. ≧ 90%.

Stages b) + c)

1 ml of a buffer solution consisting of Na phosphate at pH = 7, 3.3 g of isobutyl R(+)-3,4-epoxybutyrate (20.9 mmoles) and 30 mg of enzyme subtilisina BPN′ (sold by the firm SIGMA Chemical Co. USA, having an activity of 6-9 units per mg of solid), were added to 30 ml of water. The mixture was stirred strongly at 20 °C, and the pH was kept constant at value 7 by addition of an aqueous solution of 1N NaOH.

After 70 hours the reaction mixture was extracted with methylene chloride and 5 ml of an aqueous solution of 5N trimethylamine were added to the aqueous phase. The resulting solution was brought to 45 °C for two hours, under strong stirring.

At the end, the solution was evaporated under reduced pressure (about 20 mm Hg). A sample of the residue, analysed at NMR in $D_2O$), proved to consist of compound (IV). The residue was dissolved again with 3 ml of concentrated aqueous hydrochloric acid. Then the solution was evaporated again under reduced pressure and the residue was treated with ethanol. The resulting mixture was brought to the boiling temperature over 20 minutes and filtered. 3.28 g of L(-)-carnitine chloride, $[\alpha]_D^{20}$ = -21.3° (c = 1, $H_2O$), e.e. 90%, were crystallized from the filtrate, by cooling.

EXAMPLE 2

Stage a)

One operated as in example 1, while replacing, however, isobutyl (R,S)-epoxybutyrate with 10 g of n-butyl (R,S)-3,4-epoxybutyrate (63.3 mmoles). After 26 hours, (ester conversion equal to 60%) 3.8 g of n-butyl R(+)-3,4-epoxybutyrate were recovered, by extraction with methylene chloride and subsequent distillation.

NMR analysis at 300 $MH_z$, in the presence of Eu $(hfc)_3$, showed an e.e. ≧ 90%.

Stages b) + c)

One operated as in example 1, while replacing, however, isobutyl R(+)-3,4-epoxybutyrate with 3.8 g of n-butyl R(+)-3,4-epoxybutyrate (24.1 mmoles). At the end, 3.8 g of L(-)-carnitine chloride, $[\alpha]_D^{20}$ = -22.0 °C (c = 1, $H_2O$), e.e. 93%, were obtained.

EXAMPLES 3-8

One operated as in example 1, while making, however, the changes indicated on Table 1. The obtained results are indicated on Table 1 as well.

TABLE I

| EXAMPLE No. | R | STAGE a) ENZYME (amount) | STAGE a) TIME (hours) | STAGE a) ESTER CONV. % | STAGE a) % e.e. ESTER RESIDUE | STAGE b) ENZYME (amount) | STAGE b) TEMP. | STAGE b) TIME (hours) | g. of CARNITINE CHLORIDE | e. e. % CARNIT. CHLORIDE |
|---|---|---|---|---|---|---|---|---|---|---|
| 3 | iso-butyl | Lipase from Candida Cylindracea (400 mg) | 39 | 57% | 74% | Subtilisina Carlsberg (50 mg) | 20° | 12 | 4.0 | 75% |
| 4 | iso-butyl | Esterase from swine liver (3.5 mg) | 8 | 52% | 62% | Subtilisina Carlsberg (50 mg) | 20° | 12 | 4.5 | 64% |
| 5 | iso-butyl | Pancreatin (240 mg) | 21 | 60% | 75% | Subtilisina BPN' (30 mg) | 20° | 70 | 3.7 | 73% |
| 6 | n-octyl | Steapsin (500 mg) | 24 | 60% | 95% | Alcalase ® L (400 mg) | 20° | 15 | 2.8 | 95% |
| 7 | methyl | Lipase from Candida Cylindracea (400 mg) | 48 | 60% | 50% | Alcalase ® L (400 mg) | 20° | 15 | 5.1 | 51% |
| 8 | benzyl | Steapsin (500 mg) | 6.5 | 62% | 58% | Alcalase ® T (200 mg) | 40° | 18 | 2.9 | 60% |

EXAMPLE 9

Stage a)

The content of a slant of Arthrobacter simplex (IFO 3530) was inoculated in 50 ml of "Nutrient Broth" (nutrient broth manufactured by the firm Oxoid Ltd. U.K.) and kept under stirring at 200 rounds per minute, at 37°C, over 18 hours in a 250 ml flask. Then four ml of this medium were inoculated in 100 ml of "Nutrient Broth" and kept under stirring at 200 rounds per minute, at 37°C, in a 500 ml flask.

After 12 hours, 50 ml of a buffer solution consisting of K phosphate at pH = 7 and 5 g of isobutyl (R,S)-3,4-epoxybutyrate were added to the resulting mixture and the whole was kept under stirring at 20°C for 48 hours. At the end the mixture was extracted with methylene chloride, the solvent was evaporated and residual isobutyl 3,4-epoxybutyrate was purified by chromatography on silica column. Thus one obtained 2.25 g of isobutyl R(+)-3,4-epoxybutyrate having an e.e. = 75%.

Stages b) + c)

One operated as in example 1, thereby obtaining 2.03 g of L(-)-carnitine chloride having an e.e. = 75%.

EXAMPLE 10

One operated as in example 9, with the difference that the used microorganism was Pseudomonas fragi (IFO 3458), thereby obtaining 2.35 g of isobutyl R(+)-3,4-epoxybutyrate having an e.e. = 56%. Then from this ester one obtained 2.12 g of L(-)-carnitine chloride having an e.e. = 53%.

EXAMPLE 11

One operated as in example 9 with the difference that the used microorganism was Bacillus subtilis - (ATCC 6633), thereby obtaining 2.85 g of isobutyl R(+)-3,4-epoxybutyrate having an e.e. = 52%. Then from this ester one obtained 2.49 g of L(-)-carnitine chloride having an e.e. = 51%.

EXAMPLE 12

Stage a)

The content of a slant of Rodotorula minuta (IFO 0879) was inoculated in 50 ml of a culture medium having the following composition:
0.3% of yeast extract by the firm Oxoid Ltd. U.K.,
1% of peptone, Oxoid,
2% of glucose.

The medium was put into a 500 ml flask and kept under stirring at 160 rounds per minute, at 28°C, over 18 hours. Then four ml of this medium were drawn and inoculated in 100 ml of a culture medium having the same composition as the one described hereinbefore; then 0.5 g of calcium carbonate were added and the whole was kept under stirring at 16 rounds per minute, at 28°C. After 24 hours, 5 g of isobutyl (R,S)-3,4-epoxybutyrate were added to the resulting mixture and the whole was kept under stirring over 72 hours at 20°C. At the end, the mixture was extracted with methylene chloride, the solvent was evaporated and residual isobutyl R(+)-3,4-epoxybutyrate was purified by chromatography on silica column. Thus one obtained 3.1 g of isobutyl R(+)-3,4-epoxybutyrate having an e.e. = 27%.

Stages b) + c)

One operated as in example 1, thereby obtaining 2.8 g of L(-)-carnitine chloride having an e.e. = 28%.

EXAMPLE 13

One operated as in example 12 with the difference that the used microorganism was Candida cylindracea (ATCC 14830), thereby obtaining 2.75 g of isobutyl R(+)-3,4-epoxybutyrate having an e.e. = 47%. Then from this ester one obtained 2.5 g of L(-)-carnitine chloride having an e.e. = 45%.

## Claims

**1.** A process for the biotechnological preparation of L(-)-carnitine chloride, having formula (I):

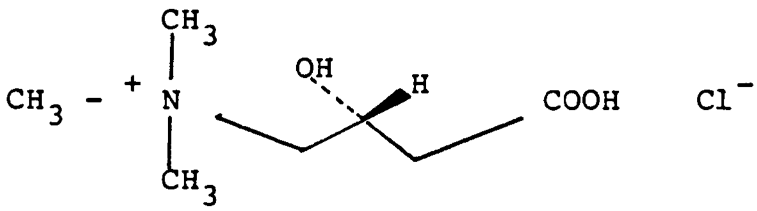

(I)

which comprises:

a) reacting a racemic ester of (R,S)-3,4-epoxybutyric acid having formula (II):

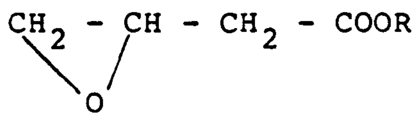

(II)

wherein R represents a $C_1$-$C_{10}$ alkyl group or a benzyl group, with an enzyme or with a microorganism producing said enzyme, capable of hydrolyzing asymmetrically and in a prevalent way enantiomer S(-), under pH conditions controlled by means of alkalis and separating said enantiomer S(-) according to usual techniques from non-reacted ester (II) which is substantially present in the R(+) form;

b) reacting the R(+) form of ester (II), thus obtained, with an enzyme capable of hydrolyzing said R-(+) form quantitatively, under pH conditions controlled by means of alkalis, to obtain a salt of 3,4-epoxybutyric acid having formula (III):

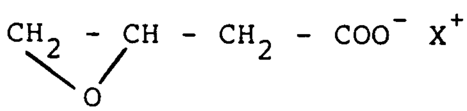

(III)

wherein X is a cation such as Na, K or Li, substantially in the R(+) form and

c) reacting said R(+) form of the salt having formula (III) with trimethylamine in a molar excess and, after having removed said excess by means of HCl, obtaining L(-)-carnitine chloride having formula (I).

**2.** A process according to claim 1, wherein the asymmetric hydrolysis of the racemic ester having formula (II) is carried out using an enzyme, preferably selected from steapsin, pancreatin and lipase from Candida Cylindracea.

**3.** A process according to claim 1, wherein the asymmetric hydrolysis of the racemic ester having formula (II) is carried out using an enzyme produced by a microorganism, preferably selected from Pseudomonas Fragi (IFO 3458), Bacillus subtilis (ATCC 6633), Rodotorula minuta (IFO 0879), Candida Cylindracea (ATCC 14830) and Arthrobacter simplex (IFO 3530).

**4.** A process according to claim 3, wherein the microorganism is used in the form of a culture broth, a filtrate, a concentrate or a suspension of its cells.

**5.** A process according to claim 2, wherein the enzyme is immobilized on a substrate.

**6.** A process according to any one of claims 1 to 5, wherein the asymmetric hydrolysis of the racemic ester (II) is carried out using the enzyme in an amount of from 0.03% to 10% by weight, based on the racemic ester (II).

**7.** A process according to any one of claims 1 to 6, wherein the asymmetric hydrolysis of the racemic ester (II) is carried out at a temperature of from 10 to 30 ° C.

**8.** A process according to any one of claims 1 to 7, wherein the asymmetric hydrolysis of the racemic ester (II) is carried out at a pH value of from 5 to 9 using a basic buffer solution or a mineral base.

**9.** A process according to any of claims 1 to 8, wherein the concentration of the racemic ester (II) in the mixture for the asymmetric hydrolysis ranges from 1 to 20% by weight.

**10.** A process according to any one of claims 1 to 9, wherein the hydrolysis of the R(+) form of the ester having formula (II) is carried out using a hydrolytic enzyme, preferably selected from Subtilisina BPN′, Subtilisina Carlsberg and Alcalase ®.

**11.** A process according to claim 10, wherein the enzyme is used immobilized on a substrate.

**12.** A process according to any one of claims 1 to 11, wherein the hydrolysis of the R(+) ester having formula (II) is carried out by using the enzyme in an amount of from 0.5 to 30% by weight, based on the R(+) ester having formula (II).

**13.** A process according to any one of claims 1 to 13, wherein the hydrolysis of the R(+) ester having formula (II) is carried out at a pH value of from 5 to 10 using a basic buffer solution or a mineral base.

**14.** A process according to any one of claims 1 to 14, wherein the concentration of the R(+) ester having formula (II) in the hydrolysis mixture ranges from 1 to 20% by weight.

**15.** A process according to any one of claims 1 to 15, wherein trimethylamine is added to the salt of R(+)-3,4-epoxybutyric acid having formula (III) in the form of an aqueous trimethylamine solution having a concentration of from 0.1 to 5.5 moles/litre in a molar ratio of from 1.5 to 3 moles per mole of the salt of R(+)-3,4-epoxybutyric acid (III) at a temperature of from 10 to 60 ° C, preferably 40 to 50 ° C.

**Revendications**

**1.** Un procédé destiné à la préparation biotechnologique de chlorure de L(-)-carnitine, ayant la formule (I):

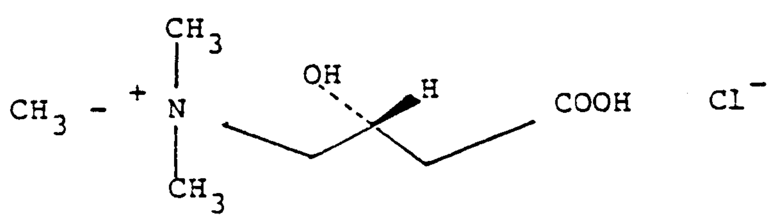

(I)

qui consiste:

a) à faire réagir un ester racémique de l'acide (R,S)-3,4-époxybutyrique ayant la formule (II):

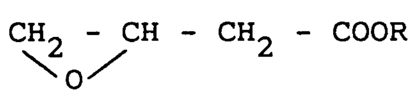

, (II)

dans laquelle R représente un groupe alkyle en $C_1$-$C_{10}$ ou un groupe benzyle, avec une enzyme ou avec un micro-organisme produisant cette enzyme, capable d'hydrolyser d'une manière asymétrique et prédominante l'énantiomère S(-), dans des conditions de pH régulées au moyen d'alcali, et à séparer, selon les techniques usuelles, ledit énantiomère S(-) de l'ester (II) n'ayant pas réagi, lequel est pratiquement présent sous forme R(+);

b) à faire réagir la forme R(+) de l'ester (II) ainsi obtenu avec une enzyme capable d'hydrolyser ladite forme R(+) d'une manière quantitative, dans des conditions de pH régulées au moyen d'alcali, pour obtenir un sel de l'acide 3,4-époxybutyrique ayant la formule (III):

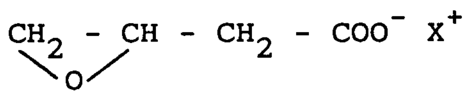

(III)

dans laquelle X est un cation tel que Na, K ou Li, pratiquement sous forme R(+), et

c) à faire réagir ladite forme R(+) du sel de formule (III) avec de la triméthylamine selon un excès molaire et, après élimination dudit excès au moyen de HCl, à obtenir du chlorure de L(-)-carnitine de formule (I).

**2.** Un procédé selon la revendication 1, dans lequel l'hydrolyse asymétrique de l'ester racémique de formule (II) est mise en oeuvre à l'aide d'une enzyme de préférence choisie parmi la stéapsine, la pancréatine et la lipase de Candida cylindracea.

**3.** Un procédé selon la revendication 1, dans lequel l'hydrolyse asymétrique de l'ester racémique de formule (II) est mise en oeuvre à l'aide d'une enzyme produite par un micro-organisme, choisi de préférence parmi Pseudomonas fragi (IFO 3458), Bacillus subtilis (ATCC 6633), Rodotorula minuta (IFO 0879), Candida cylindracea (ATCC 14830) et Arthrobacter simplex (IFO 3530).

**4.** Un procédé selon la revendication 3, dans lequel le micro-organisme utilisé se présente sous forme d'un bouillon de culture, d'un filtrat, d'un concentré ou d'une suspension de ses cellules.

**5.** Un procédé selon la revendication 2, dans lequel l'enzyme est immobilisée sur un substrat.

**6.** Un procédé selon l'une quelconque des revendications 1 à 5, dans lequel l'hydrolyse asymétrique de l'ester racémique (II) est mise en oeuvre par utilisation de l'enzyme en une proportion de 0,03 à 10% en poids par rapport à l'ester racémique II.

**7.** Un procédé selon l'une quelconque des revendications 1 à 6, dans lequel l'hydrolyse asymétrique de l'ester racémique (II) est mise en oeuvre à une température de 10 à 30 ° C.

**8.** Un procédé selon l'une quelconque des revendications 1 à 7, dans lequel l'hydrolyse asymétrique de l'ester racémique (II) est mise en oeuvre à un pH de 5 à 9, en présence d'une solution tampon basique ou d'une base minérale.

**9.** Un procédé selon l'une quelconque des revendications 1 à 8, dans lequel la concentration de l'ester racémique (II) dans le mélange destiné à l'hydrolyse asymétrique est comprise entre 1 et 20% en poids.

**10.** Un procédé selon l'une quelconque des revendications 1 à 9, dans lequel l'hydrolyse de la forme R(+) de l'ester de formule (II) est mise en oeuvre en présence d'une enzyme hydrolytique choisie de préférence parmi Subtilisina BPN', Subtilisina Carlsberg et Alcalase®.

**11.** Un procédé selon la revendication 10, dans lequel l'enzyme est utilisée immobilisée sur un substrat.

**12.** Un procédé selon l'une quelconque des revendications 1 à 11, dans lequel l'hydrolyse de l'ester R(+) de formule (II) est mise en oeuvre en présence de l'enzyme utilisé en une proportion de 0,5 à 30% en poids par rapport à l'ester R(+) de formule (II).

**13.** Un procédé selon l'une quelconque des revendications 1 à 12, dans lequel l'hydrolyse de l'ester R(+) de formule (II) est mise en oeuvre à un pH de 5 à 10, en présence d'une solution tampon basique ou d'une base minérale.

**14.** Un procédé selon l'une quelconque des revendications 1 à 13, dans lequel la concentration de l'ester R(+) de formule (II) dans le mélange d'hydrolyse est comprise entre 1 et 20% en poids.

**15.** Un procédé selon l'une quelconque des revendications 1 à 14, dans lequel la triméthylamine est ajoutée au sel de l'acide R(+)-3,4-époxybutyrique de formule (III) sous forme d'une solution aqueuse de triméthylamine ayant une concentration de 0,1 à 5,5 moles par litre, selon une proportion molaire de 1,5 à 3 moles par mole du sel de l'acide R(+)-3,4-époxybutyrique (III), à une température de 10 à 60 °C, de préférence de 40 à 50 °C.

## Patentansprüche

**1.** Verfahren zur biotechnischen Herstellung von L(-)-Carnitinchlorid der Formel (I):

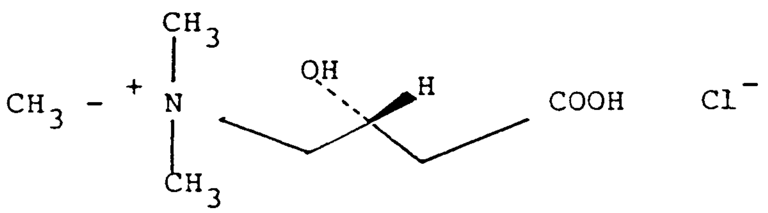 (I)

welches umfaßt:

a) das Umsetzen eines racemischen Esters von (R,S)-3,4-Epoxybuttersäure der Formel (II):

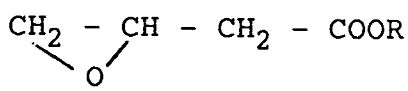 (II)

worin R eine $C_1$-$C_{10}$ Alkylgruppe oder eine Benzylgruppe darstellt, mit einem Enzym oder mit einem Mikroorganismus, der das Enzym erzeugt, das imstande ist, asymmetrisch und in bevorzugter Weise das s(-)-Enantiomer zu hydrolysieren, unter pH-Bedingungen, die mit Alkali kontrolliert werden, und die Abtrennung des s(-)- Enantiomers mit üblichen Methoden von nicht umgesetztem Ester (II), der im wesentlichen in der R(+)-Form vorliegt;

b) das Umsetzen der so erhaltenen R(+)-Form des Esters (II) mit einem Enzym, das imstande ist, die R(+)-Form quantitativ zu hydrolysieren, unter pH-Bedingungen, die mit Alkali kontrolliert werden, um ein Salz von 3,4-Epoxybuttersäure der Formel (III) :

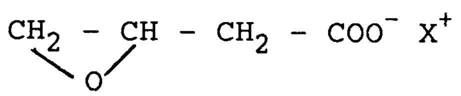 (III)

worin X ein Kation wie z.B. Na, K oder Li ist, im wesentlichen in der R(+)-Form zu erhalten, und

c) das Umsetzen der R(+)-Form des Salzes der Formel (III) mit Trimethylamin in molarem Überschuß und nach dem Entfernen des Überschusses mit HCl, das Erhalten von L(-)-Carnitinchlorid der Formel (I).

**2.** Verfahren nach Anspruch 1, worin die asymmetrische Hydrolyse des racemischen Esters der Formel (II) unter Verwendung eines Enzyms ausgeführt wird, das vorzugsweise aus Steapsin, Pankreatin und Lipase von Candida Cylindracea ausgewählt ist.

**3.** Verfahren nach Anspruch 1, worin die asymmetrische Hydrolyse des racemischen Esters der Formel (II) unter Verwendung eines Enzyms ausgeführt wird, das von einem Mikroorganismus erzeugt wird, der vorzugsweise aus Pseudomonas Fragi (IFO 3458), Bacillus subtilis (ATCC 6633), Rodotorula minuta - (IFO 0879), Candida Cylindracea (ATCC 14830) und Arthrobacter simplex (IFO 3530) ausgewählt ist.

**4.** Verfahren nach Anspruch 3, worin der Mikroorganismus in Form einer Kulturflüssigkeit, eines Filtrats, eines Konzentrats oder einer Suspension seiner Zellen verwendet wird.

**5.** Verfahren nach Anspruch 2, worin das Enzym auf einem Träger immobilisiert ist.

**6.** Verfahren nach irgendeinem der Ansprüche 1 bis 5, worin die asymmetrische Hydrolyse des racemischen Esters (II) unter Verwendung des Enzyms in einer Menge von 0,03 bis 10 Gew.-%, bezogen auf den racemischen Ester (II), durchgeführt wird.

**7.** Verfahren nach irgendeinem der Ansprüche 1 bis 6, worin die asymmetrische Hydrolyse des racemischen Esters (II) bei einer Temperatur von 10 bis 30 ° C durchgeführt wird.

**8.** Verfahren nach irgendeinem der Ansprüche 1 bis 7, worin die asymmetrische Hydrolyse des racemischen Esters (II) bei einem pH-Wert von 5 bis 9 unter Verwendung einer basischen Pufferlösung oder einer Mineralbase durchgeführt wird.

**9.** Verfahren nach irgendeinem der Ansprüche 1 bis 8, worin die Konzentration des racemischen Esters (II) in der Mischung für die asymmetrische Hydrolyse im Bereich von 1 bis 20 Gew.-% liegt.

**10.** Verfahren nach irgendeinem der Ansprüche 1 bis 9, worin die Hydrolyse der R(+)-Form des Esters der Formel (II) unter Verwendung eines hydrolytischen Enzyms durchgeführt wird, das vorzugsweise aus Subtilisina BPN', Subtilisina Carlsberg und Alcalase [(R)] ausgewählt ist.

**11.** Verfahren nach Anspruch 10, worin das Enzym auf einem Träger immobilisiert verwendet ist.

**12.** Verfahren nach irgendeinem der Ansprüche 1 bis 11, worin die Hydrolyse des R(+)-Esters der Formel (II) durchgeführt wird unter Verwendung des Enzyms in einer Menge von 0,5 bis 30 Gew.-%, bezogen auf den R(+)-Ester der Formel (II).

**13.** Verfahren nach irgendeinem der Ansprüche 1 bis 12, worin die Hydrolyse des R(+)-Esters der Formel (II) bei einem pH-Wert von 5 bis 10 unter Verwendung einer basischen Pufferlösung oder einer Mineralbase durchgeführt wird.

**14.** Verfahren nach irgendeinem der Ansprüche 1 bis 13, worin die Konzentration des R(+)-Esters der Formel (II) in der Hydrolysemischung 1 bis 20 Gew.-% beträgt.

**15.** Verfahren nach irgendeinem der Ansprüche 1 bis 14, worin Trimethylamin zum Salz der R(+)-3,4-Epoxybuttersäure der Formel (III) in Form einer wäßrigen Trimethylaminlösung mit einer Konzentration von 0,1 bis 5,5 Mol/l in einem Molverhältnis von 1,5 bis 3 Mol pro Mol des Salzes der R(+)-3,4-Epoxybuttersäure (III) und bei einer Temperatur von 10 bis 60 ° C, vorzugsweise 40 bis 50 ° C, zugegeben wird.